Europäisches Patentamt

(19) European Patent Office

Office européen des brevets

(11) Publication number: **0 074 291**
**B1**

(12) **EUROPEAN PATENT SPECIFICATION**

(45) Date of publication of patent specification: **26.02.86**

(21) Application number: **82401483.1**

(22) Date of filing: **05.08.82**

(51) Int. Cl.⁴: **A 61 M 1/02**

(54) **Air purge unit for autotransfusion apparatus and autotransfusion apparatus comprising the same.**

(30) Priority: **05.08.81 US 290666**
**14.08.81 US 292425**

(43) Date of publication of application:
**16.03.83 Bulletin 83/11**

(45) Publication of the grant of the patent:
**26.02.86 Bulletin 86/09**

(84) Designated Contracting States:
**DE FR GB IT**

(56) References cited:
**US-A-3 993 067**
**US-A-4 111 204**
**US-A-4 228 798**

(73) Proprietor: **BIORESEARCH INC.**
**315 Smith Street**
**Farmingdale, NY 11735 (US)**

(72) Inventor: **Kurtz, Leonard D.**
**46 Woodmere Boulevard**
**Woodmere New York (US)**
Inventor: **LiCausi, Joseph**
**43 Penn Street**
**Port Jefferson Station New York (US)**

(74) Representative: **Ayache, Monique et al**
**CABINET PLASSERAUD 84, rue d'Amsterdam**
**F-75009 Paris (FR)**

Courier Press, Leamington Spa, England.

Description

This invention relates generally to an autotransfusion device and more specifically to an air purge unit for an autotransfusion device which is adapted to be mounted on the collection chamber for blood of the autotransfusion device.

During certain types of surgery, particularly in connection with chest cavity surgery, the patient frequently loses large amounts of blood. Ordinarily, the lost blood is aspirated away and the patient is given a transfusion of donated blood to make up for the lost blood. An alternative arrangement is to provide an autotransfusion device which will collect the blood lost by the patient and transfuse this blood into the patient's circulatory system. Such an apparatus is disclosed in the copending European Patent Application EP—A—0 072 738.

There are disclosed in the prior art various systems for autotransfusion. For example, in US—A—3,191,600, an autotransfusion apparatus is disclosed which includes a vacuum source and a plurality of suction tips for immersion in pools of blood. The blood is collected in a collection chamber and is returned to the patient through a one-way valve. Another autotransfusion device is disclosed in US—A—3,492,991 and includes a container equipped with a filter through which the blood is gravity fed back to the patient. In US—A—3,993,067, an autotransfusion device is disclosed in which the blood is forced back into the patient by pressure in the collection chamber. Still another autotransfusion device is disclosed in US—A—4,047,526. This patent discloses a collection chamber in which blood is continuously aspirated. A blood bag with an outwardly urged spring is connected to the collection chamber to withdraw some of the blood therefrom. The blood collected in the blood bag is then later reintroduced into the patient. There has also been disclosed in the prior art a spring operated device which forces blood from a blood bag into the patient. Such a device is disclosed in US—A—3,565,292.

While autotransfusion has been disclosed in the prior art, there is a need for an air purge unit which will effectively prevent the possibility of air being reintroduced into the patient's circulatory system which could lead to disastrous consequences.

US—A—4,111,204 describes a suction collection system having at its upper end an air purge unit which comprises a housing having a passageway therein, a filter of air pervious liquid-impervious material and, in series with said filter, a oneway valve, which is a float valve designed to prevent the flow of liquid out of the collection system. Such an air purge unit is not adapted to effectively prevent the possibility of air being reintroduced into the patient's circulatory system through an autotransfusion device.

According to the present invention, an air purge unit is provided which ensures that all air within the collection chamber for the blood is eliminated before the blood is reintroduced into the patient's circulatory system.

In accordance with the present invention, an air purge unit is provided which is adapted to be mounted on the top wall of a collection chamber for blood in an autotransfusion apparatus such as disclosed in the European patent application EP—A—0 072 738.

More precisely, the invention provides an air purge unit to be mounted on the collection chamber for blood of an autotransfusion apparatus comprising, in combination, a housing having a passageway therein, a filter having a pore size smaller than the size of blood cells and a oneway valve, characterised in that said oneway valve is designed to prevent reverse flow of air into the collection chamber and is in series with said filter across said passageway, and the combination is adapted so that when air and blood from the collection chamber are forced through the passageway of the housing, air passes through said filter and through said oneway valve and blood blocks said filter to close said passageway.

The invention also provides an autotransfusion apparatus comprising a collection chamber for blood and an air purge as defined above.

According to a preferred embodiment, the invention provides an autotransfusion apparatus comprising a collection chamber for blood and an air purge unit directly mounted on said collection chamber for purging the air within the collection chamber when blood and air within the collection chamber are put under positive pressure, said air purge unit comprising a housing, a passageway in said housing and having an outlet in its upper part opposite to the collection chamber, a filter disposed in said passageway, said filter having a pore size smaller than the size of red blood cells and a oneway valve in series with the filter, characterised in that:

— said passageway is adapted to be in fluid communication with the interior of said collection chamber;

— said filter permits air to pass therethrough until blood fills said passageway between the interior of said collection chamber and the filter; and

— said oneway valve is disposed across the passageway, between said filter and said outlet and is designed to permit air to pass therethrough from the interior of said collection chamber and to prevent reverse flow of air into the collection chamber.

The air purge unit comprises a housing having a passageway therethrough with the inner end of the passageway being in fluid communication with the interior of the collection chamber. There is provided a filter within the passageway which has a pore size smaller than the diameter of red blood cells. Thus, this filter will pass any fluids having particles of a size smaller than the pore size of the filter, but will not permit red blood cells to pass therethrough. Adjacent the filter within the passageway, there is provided an umbrella type valve which will open to permit air or fluids to pass therethrough but, which will prevent the admission of outside air from entering the interior of the collection chamber. On the outlet from the housing is a flap valve which is also a oneway valve and also serves to ensure that no atmospheric air can enter the air purge unit.

In use, a tube connects the air purge unit with a source of regulated suction so that any air which is drawn into the collection chamber is withdrawn through the purge unit. As more fully disclosed in applicant's copending application, when the collection chamber is filled with blood the collection chamber is put under pressure so as to force any air remaining within the collection chamber out through the air purge unit. When the passage way between the filter and the interior of the collection chamber is filled with blood, the red blood cells clog the filter to block the filter to prevent the loss of blood therethrough. Thus, all air within the collection chamber is removed and the possibility of an air emboli within the patient's circulatory system is virtually eliminated.

Other features and advantages of the present invention are stated or are apparent from the detailed description of a presently preferred embodiment of the invention found hereinbelow.

In the drawings:

Figure 1 is a perspective view showing an autotransfusion system with an air purge unit according to the present invention installed on the collection chamber,

Figure 2 is a cross-sectional view of the air purge unit,

Figure 3 is an exploded view of the parts of the air purge unit shown in Figure 2,

Figure 4 is a cross-sectional view of a modified form of air purge unit, and

Figure 5 is an exploded view of the parts of the air purge unit shown in Figure 4.

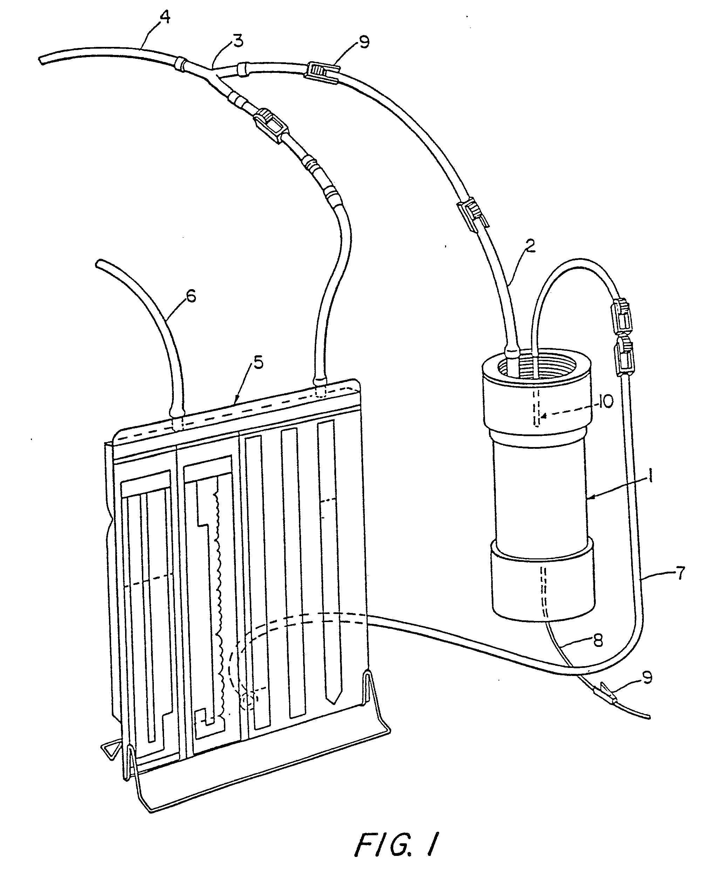

With reference now to the drawings in which like numerals represent like parts throughout the several views, a perspective view of the autotransfusion system is shown in Figure 1 wherein there is shown at 1 a collection chamber for blood. This collection chamber is disclosed in detail in copending European patent application EP—A—0 072 738. The inlet to the collection chamber is shown at 2 and connects through a Y connector 3 with a tube 4, the free end of which is disposed in a patient's body cavity to collect blood therefrom. A three chambered drainage apparatus is shown at 5, this device being similar to the underwater drainage device disclosed in US—A—3,363,626 and US—A—3,363,627. Such an underwater drainage device will maintain any degee of suction in accordance with the setting in the manometer chamber in the device. The tube 6 extending from the underwater drainage device is connected with a source of suction. A tube 7 extends from an outlet in the upper end of the collection chamber and is connected with the underwater drainage device so that the regulated negativity maintained by the underwater drainage device passed through the tube 7 into the interior of the collection chamber 1.

An outlet 8 extends from the bottom wall of the collection chamber. Clamps such as shown at 9 are located on tubes 2, 7 and 8. As described in the copending application hereinbefore referred to, the device is operated by applying suction through tube 6 so as to aspirate blood through the tubes 4 and 2 into the collection chamber. Air which is within the collection chamber is drawn out through tube 7. When the collection chamber is filled with blood, the locking clamp on tube 2 is closed and the spring mechanism on the collection chamber is released so as to force the top wall of the chamber downwardly in the manner described in the hereinbefore referred to copending patent application. Any air remaining within the upper end of the collection chamber is forced out through tube 7. Upon completion of the air purge from the collection chamber, the locking clamp on tube 7 is closed and the clamp on tube 8 is opened so that blood is forced outwardly through the tube 8 into the circulatory system of the patient.

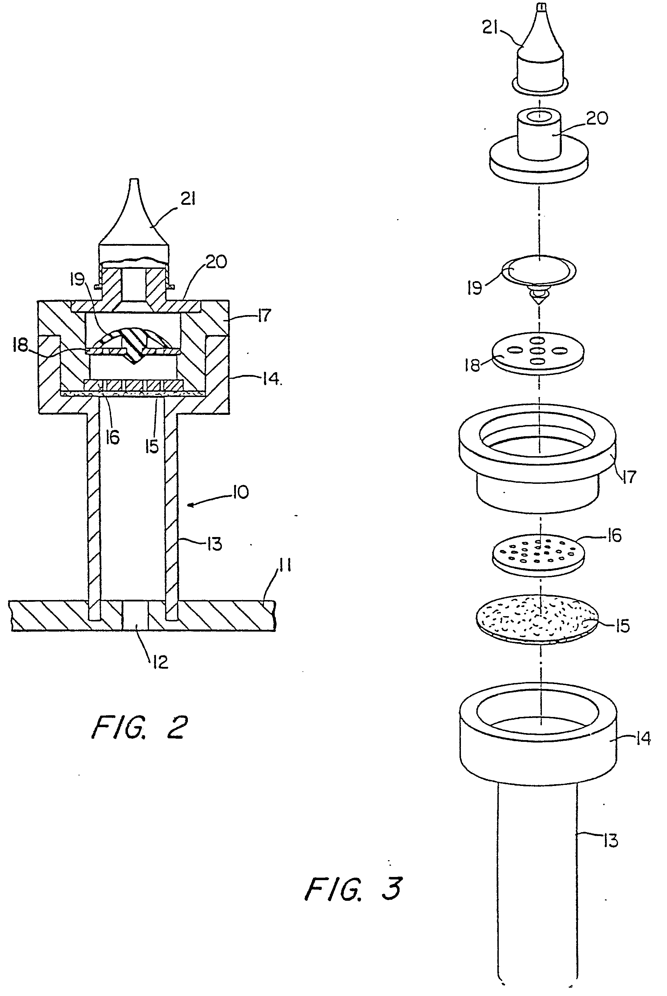

The air purge unit according to the present invention is disposed as shown at 10 in Figure 1 and is connected with the tube 7 so as to be disposed within the pathway between the interior of the collection chamber and the suction source. The top wall 11 of the collection chamber is shown in Figure 2 as having an outlet aperture 12 disposed therein. The air purge unit 10 is located on the top wall and comprises a housing 13 with a passageway extending therethrough and having a flanged upper end portion as shown at 14. Seated on the horizontal outwardly extending surface of the flange 14 is a filter element 15. This filter element has a pore size of approximately two microns so that air may readily diffuse through this filter. Disposed on the upper surface of the filter is a spacer element 16 having a plurality of apertures therein. The filter and spacer are held in position by means of a valve seat housing 17 which is press fit within the outer flange portion 14 of housing element 13. Seated within member 17 is an umbrella type apertured valve seat 18 having a oneway resilient umbrella valve 19 mounted thereon. An apertured plug insert 20 is seated within a recess in the upper end of the valve seat housing 17 and a resilient oneway flap valve 21 is fitted over the outer surface of the plug insert 20.

The filter 15 having a pore size of two microns will permit any air within the collection chamber to pass therethrough but will not pass red blood cells which have a size of approximately seven microns. Thus, when the passageway beneath the filter 15 becomes filled with blood, the filter prevents passage of the blood therethrough, thus blocking the air purge unit.

The one-way valve 19 and 21 ensure that no air can pass from the exterior of the unit into the collection chamber. Thus, even in the event the tube 7 becomes disconnected from the underwater drainage device or the drainage device does not function properly so that the tube 7 becomes exposed to atmospheric air, no air will be admitted into the collection chamber.

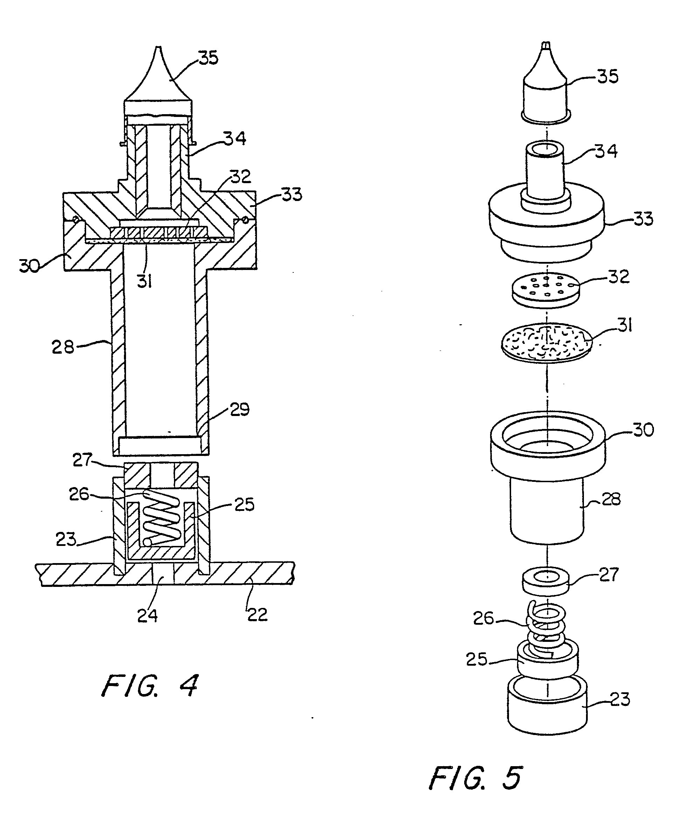

In Figures 4 and 5 there is shown a modified form of air purge unit. In this embodiment the blood bag cover 22 has a separate passageway (not shown) for connection with the suction device. The unit comprises a hollow ring shaped base 23 which is secured to the top of the blood bag cover and surrounds the opening 24.

Disposed within the base 23 is a spring housing 25 which also serves as a valve element for closing opening 24 as will appear more clearly hereinafter. A compression spring 26 is disposed within the housing 25 and acts between the housing 25 and an apertured retaining ring 27.

A lower housing member 28 of clear plastic is secured to the base 23 and an inner flange 29 forms a seat for the retaining ring 27. It can be seen that the compression spring 26 will force the retaining ring 27 and spring housing 25 apart so that normally the ring is seated on the flange 29 and the end of the housing is seated firmly against the top of the blood bag cover, thus closing the opening 24.

The upper end of the housing 28 has an outwardly extending peripheral flange portion 30 which forms a seat for a filter element 31. This filter element is similar to the element 15 of the Figures 1 to 3 embodiment and has a pore size of less than 7 microns so as to pass particles of a smaller size but to block the passage of red blood particles having a pore size of seven microns. A vented spacer support for the filter is shown at 32 which seats in a circular recess formed in the top cover 33 of the unit. The top cover is press fit within the flanged portions of lower housing member 28 so as to firmly seat the vented spacer support 32 and filter element 31.

An outlet port 34 formed in the top cover 33 is closed by a one-way flap valve 35 of the Hemlich type comprising a pair of resilient elements having a slit therebetween and normally urged together. Upon internal pressure in excess of the force holding the resilient elements together the flap valves will open and permit the passage of fluids therebetween.

In operation the spring pressed valve 25 and the flap valve 35 normally remain closed during the filling of the blood bag. After the blood bag is filled to the extent desired, the spring mechanism described in EP—A—0 072 738 filed on August 5, 1982, is released applying pressure to the contents of the bag. The internal pressure exceeds $6,65.10^{-2}$ bar (50 millimeters of mercury) and the spring valve 25 is forced open to pass air and liquid therethrough. The filter 31 will permit the passage of air therethrough as well as any particles of less than seven microns size. The flap valve 35 will open to permit the passage of air therethrough. When the filter 31 becomes clogged with red blood cells it prevents the passage of blood therethrough. The attending physician can readily determine that the air purge unit is operative by viewing the blood filled housing element 28.

Thus, while the invention has been described in detail with respect to an exemplary embodiment thereof, it will be understood by those of ordinary skill in the art that these and other variations and modifications may be effected in the exemplary embodiment within the scope of the invention as defined in the appended claims.

**Claims**

1. An air purge unit (10) to be mounted on the collection chamber (1) for blood of an autotransfusion apparatus comprising, in combination, a housing (13, 14, 17, 20 or 23, 28, 30, 33, 34) having a passageway therein, a filter (15, 31) having a pore size smaller than the size of blood cells and a oneway valve (19, 25), characterized in that: said oneway valve (19, 25) is designed to prevent reverse flow of air into the collection chamber (1) and is in series with said filter (15, 31) across said passageway, and the combination is adapted so that when air and blood from the collection chamber (1) are forced through the passageway of the housing, air passes through said filter (15, 31) and through said oneway valve (19, 25) and blood blocks said filter to close said passageway.

2. An air purge unit according to claim 1, chracterised in that it further comprises, on the top (20, 34) of the housing, opposite to the end (13, 23) to be mounted on the collection chamber (1), a oneway flap valve (21, 35) which is in series with said passsgeway of the housing.

3. An air purge unit according to claim 1 or 2, characterised in that it further includes a vented spacer (16, 32) disposed on one face of said filter.

4. An air purge unit according to any one of claims 1 to 3, characterised in that said filter (15, 31) has a pore size less than seven microns.

5. An air purge unit according to any one of claims 2 to 4, characterised in that said oneway valve (19) is an umbrella valve.

6. An air purge unit according to any one of claims 2 to 4, chracterised in that said oneway valve (25) is a spring valve adapted for normally closing said passageway and opening in response to pressure from air within the collection chamber.

7. An autotransfusion apparatus comprising a collection chamber (1) for blood and an air purge unit (10) according to any one of claims 1 to 6 for purging the air within said collection chamber.

8. An autotransfusion apparatus comprising a collection chamber (1) for blood and an air purge unit (10) directly mounted on said collection chamber (1) for purging the air within the collection chamber (1) when blood and air within the collection chamber (1) are put under positive pressure, said air purge unit comprising a housing (13, 14, 17, 20) a passageway in said housing and having an outlet in its upper part opposite to the collection chamber (1), a filter (15) disposed in said passageway, said filter having a pore size smaller than the size of red blood cells and a oneway valve (19) in series with the filter, characterised in that:

— said passageway is adapted to be in fluid communication with the interior of said collection chamber (1);

— said filter (15) permits air to pass therethrough until blood fills said passageway between the interior of said collection chamber (1) and the filter (15); and

— said oneway valve (19) is disposed across the passageway, between said filter (15) and said outlet and is designed to permit air to pass therethrough from the interior of said collection chamber (1) and to prevent reverse flow of air into the collection chamber (1).

9. An autotransfusion apparatus according to claim 8, characterised in that said oneway valve (19) is an umbrella valve and in that the purge unit (10) further includes a oneway flap valve (21) in its upper part opposite to the collection chamber (1), in series with said filter (15) and said oneway valve (19).

10. An autotransfusion apparatus according to claim 8 or 9, characterised in that said filter (15) has a pore size less than seven microns.

**Revendications**

1. Unité de purge d'air (10) destinée à être montée sur la chambre collectrice (1) de sang d'un appareil d'autotransfusion comprenant, en combinaison, un logement (13, 14, 17, 20 ou 23, 28, 30, 33, 34) dans lequel est pratiqué un passage, un filtre (15, 31) dont la dimension des pores est inférieure à celle des globules du sang et une soupape de retenue (19, 25), caractérisée en ce que ladite soupape de retenue (19, 25) est conçue pour éviter le courant inverse de l'air dans la chambre collectrice (1) et est en série avec ledit filtre (15, 31) au travers dudit passage, et en ce que la combinaison est adaptée pour que lorsque l'air et le sang provenant de la chambre collectrice (1) sont refoulés dans le passage du logement, l'air traverse ledit filtre (15, 31) et ladite soupape de retenue (19, 25) et le sang bloque ledit filtre de manière à fermer ledit passage.

2. Unité de purge d'air selon la revendication 1, caractérisée en ce qu'elle comprend en outre, à la partie supérieure (20, 34) du logement, à l'opposé de l'extrémité (13, 23) qui doit être montée sur la chambre collectrice (1), une soupape de retenue à clapet (21, 35) qui est en série avec ledit passage du logement.

3. Unité de purge d'air selon la revendication 1 ou 2, caractérisée en ce qu'elle comprend en outre un élément d'écartement ajouré (16, 32) disposé sur une face dudit filtre.

4. Unité de purge d'air selon l'une quelconque des revendications 1 à 3, caractérisée en ce que ledit filtre (15, 31) comprend des pores dont les dimensions sont inférieures à sept microns.

5. Unité de purge d'air selon l'une quelconque des revendications 2 à 4, caractérisée en ce que ladite soupape de retenue (19) est une soupape en ombrelle.

6. Unité de purge d'air selon l'une quelconque des revendications 2 à 4, caractérisée en ce que ladite soupape de retenue (25) est une soupape à ressort adaptée pour fermer normalement ledit passage et pour s'ouvrir en réponse à la pression provenant de l'air contenu dans la chambre collectrice.

7. Appareil d'autotransfusion comprenant une chambre collectrice (1) pour le sang et une unité de purge d'air (10) selon l'une quelconque des revendications 1 à 6, pour purger l'air contenu dans ladite chambre collectrice.

8. Appareil d'autotransfusion comprenant une chambre collectrice (1) pour le sang et une unité de purge d'air (10) montée directement sur ladite chambre collectrice (1) pour purger l'air contenu dans la chambre collectrice (1) quand le sang et l'air se trouvant dans la chambre collectrice (1) sont soumis à une pression positive, ladite unité de purge d'air comprenant un logement (13, 14, 17, 20), un passage dans ledit logement et une sortie à sa partie supérieure à l'opposé de la chambre collectrice (1), un filtre (15) monté dans ledit passage, ledit filtre ayant des pores de dimension inférieure à celle des globules rouges du sang et une soupape de retenue (19) en série avec le filtre, caractérisé en ce que:

— ledit passage est adapté pour être en communication fluidique avec l'intérieur de ladite chambre collectrice (1),

— ledit filtre (15) permet à l'air de le traverser jusqu'à ce que le sang remplisse ledit passage entre l'intérieur de ladite chambre collectrice (1) et le filtre (15), et

— ladite soupape de retenue (19) est montée au travers dudit passage, entre ledit filtre (15) et ladite sortie et est conçue pour permettre à l'air de la traverser depuis l'intérieur de ladite chambre collectrice (1) et pour empêcher un courant en sens inverse de l'air dans la chambre collectrice (1).

9. Appareil d'autotransfusion selon la revendication 8, caractérisé en ce que ladite soupape de retenue (19) est une soupape en ombrelle et en ce que l'unité de purge d'air (10) comprend en outre une soupape de retenue à clapet (21) à sa partie supérieure opposée à la chambre collectrice (1), en série avec ledit filtre (15) et ladite soupape de retenue (19).

10. Appareil d'autotransfusion selon la revendication 8 ou 9, caractérisé en ce que ledit filtre (15) a des pores dont les dimensions sont inférieures à sept microns.

**Patentansprüche**

1. Entlüftungseinheit (10), die auf die Blut-Sammelkammer (1) einer Autotransfusionsvorrichtung montierbar ist und in Kombination ein Gehäuse (13, 14, 17, 20 oder 23, 28, 30, 33, 34) mit einem darin ausgebildeten Durchlaß, ein Filter (15, 31) mit kleinerer Porengröße als die Größe der Blutzellen und ein Einwegventil (19, 25) aufweist, dadurch gekennzeichnet, daß das Einwegventil (19, 25) dazu bestimmt ist eine Rückströmung der Luft in die Sammelkammer (1) zu verhindern und mit dem Filter (15, 31) über den Durchlaß in Reihe geschaltet ist, und daß die Kombination derart ausgebildet ist, daß Luft durch das Filter (15, 31) und durch das Einwegventil (19, 25) fließt und Blut das Filter blockiert und den Durchlaß schließt, wenn Luft und Blut von der Sammelkammer (1) durch den Durchlaß des Gehäuses gedrückt werden.

2. Entlüftungseinheit nach Anspruch 1, ferner gekennzeichnet durch ein in Reihe mit dem Durchlaß des Gehäuses geschaltetes Einweg-Klappenventil (21, 35), das auf der Spitze (20, 34) des Gehäuses dem auf die Sammelkammer (1) montierbaren Ende (13, 23) gegenüberliegend angeordnet ist.

3. Entlüftungseinheit nach Anspruch 1 oder 2, ferner gekennzeichnet durch ein entlüftetes Abstandsstück (16, 32), das auf einer Fläche des Filters angeordnet ist.

4. Entlüftungseinheit nach einem der Ansprüche 1 bis 3, dadurch gekennzeichnet, daß die Porengröße des Filters (15, 31) weniger als 7 µm beträgt.

5. Entlüftungseinheit nach einem der Ansprüche 2 bis 4, dadurch gekennzeichnet, daß das Einwegventil (19) ein Schirmventil ist.

6. Entlüftungseinheit nach einem der Ansprüche 2 bis 4, dadurch gekennzeichnet, daß das Einwegventil (25) ein Federventil ist, das normalerweise den Durchlaß schließt und als Antwort auf einen von der Luft innerhalb der Sammelkammer ausgeübten Druck öffnet.

7. Autotransfusionsvorrichtung mit einer Blut-Sammelkammer (1) und einer Entlüftungseinheit (10) nach einem der Ansprüche 1 bis 6 zum Auslassen der Luft in der Sammelkammer.

8. Autotransfusionsvorrichtung mit einer Blut-Sammelkammer (1) und einer direkt auf der Sammelkammer (1) montierten Entlüftungseinheit (10) zum Auslassen der Luft innerhalb der Sammelkammer (1), wenn Blut und Luft innerhalb der Sammelkammer (1) unter Überdruck gesetzt werden, wobei die Entlüftungseinheit ein Gehäuse (13, 14, 17, 20), einen Durchlaß im Gehäuse und einen Auslaß in seinem der Sammelkammer (1) gegenüberliegenden Oberteil aufweist und im Durchlaß ein Filter (15) angeordnet ist, dessen Porengröße kleiner ist als die Größe der roten Blutzellen und das mit einem Einwegventil (19) in Reihe geschaltet ist, dadurch gekennzeichnet, daß

— der Durchlaß in Strömungsverbindung mit dem Innern der Sammelkammer (1) steht;

— das Filter (15) Luft durchläßt, bis Blut den Durchlaß zwischen dem Innern der Sammelkammer (1) und dem Filter (15) füllt; und

— das Einwegventil (19) im Durchlaß zwischen dem Filter (15) und dem Auslaß angeordnet ist und dazu bestimmt ist Luft vom Innern der Sammelkammer (1) durchzulassen und eine Rückströmung der Luft in die Sammelkammer (1) zu verhindern.

9. Autotransfusionsvorrichtung nach Anspruch 8, dadurch gekennzeichnet, daß das Einwegventil (19) ein Schirmventil ist und daß die Entlüftungseinheit (10) ferner ein Einweg-Klappenventil (21) in seinem der Sammelkammer (1) gegenüberliegenden Oberteil aufweist, das in Reihe mit dem Filter (15) und dem Einwegventil (19) geschaltet ist.

10. Autotransfusionsvorrichtung nach Anspruch 8 oder 9, dadurch gekennzeichnet, daß das Filter (15) eine Porengröße von weniger als 7 µm aufweist.

FIG. 1

FIG. 2

FIG. 3

2

FIG. 4

FIG. 5